(19) Europäisches Patentamt
European Patent Office
Office européen des brevets

(11) Veröffentlichungsnummer: **0 041 615**
**B1**

# (12) EUROPÄISCHE PATENTSCHRIFT

(45) Veröffentlichungstag der Patentschrift: **22.02.84**

(51) Int. Cl.³: **A 61 K 31/415** // C07D233/61, C07D233/60

(21) Anmeldenummer: **81103479.2**

(22) Anmeldetag: **07.05.81**

(54) **Verwendung von Imidazolyl-vinyl-ketonen und-carbinolen als antimikrobielle Mittel.**

(30) Priorität: **19.05.80 DE 3019027**

(43) Veröffentlichungstag der Anmeldung: **16.12.81 Patentblatt 81/50**

(45) Bekanntmachung des Hinweises auf die Patenterteilung: **22.02.84 Patentblatt 84/8**

(84) Benannte Vertragsstaaten: **BE CH DE FR GB IT LI NL SE**

(56) Entgegenhaltungen:
**DE - A - 2 738 640**
**GB - A - 2 004 276**
**US - A - 4 182 862**

(73) Patentinhaber: **BAYER AG, Zentralbereich Patente, Marken und Lizenzen, D-5090 Leverkusen 1, Bayerwerk (DE)**

(72) Erfinder: **Elbe, Hans-Ludwig, Dr., Dasnoeckel 59, D-5600 Wuppertal 1 (DE)**
Erfinder: **Büchel, Karl Heinz, Prof. Dr., Dabringhausener Strasse 42, D-5093 Burscheid (DE)**
Erfinder: **Plempel, Manfred, Dr., Pahlkestrasse 5, D-5600 Wuppertal 1 (DE)**

**EP 0 041 615 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verwendung von Imidazolyl-vinyl-ketonen und -carbinolen als antimikrobielle Mittel

Die vorliegende Erfindung betrifft die Verwendung von neuen Imidazolyl-vinyl-ketonen und -carbinolen als antimikrobielle Mittel, insbesondere als Antimykotika.

Es ist bereits bekannt geworden, daß bestimmte 1-Phenyl-2-imidazolyl-4,4-dimethyl-1-penten-3-one und -ole gute fungizide Wirksamkeit besitzen (vgl. DE-OS 2 838 847). Die Wirkung dieser Verbindungen gegen humanpathogene Pilze ist jedoch nicht immer voll befriedigend, insbesondere auch ihre in-vivo-Wirkung. Das gleiche gilt für die aus DE-A 2 738 640 bekannten Verbindungen der Formel

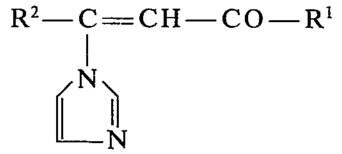

und für die aus GB-A 2 004 276 bekannten Verbindungen der Formel

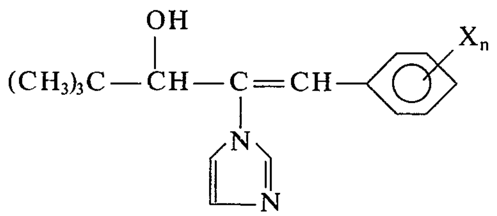

Aus US-A 4 182 862 ist ein Verfahren zur Herstellung von Azolderivaten bekannt, die jedoch als Mittel zur Bekämpfung von phytopathogenen Pilzen, nicht aber als Antimykotika verwendet werden sollen.

Es wurde gefunden, daß die neuen Imidazol-vinyl-ketone und/oder Carbinole der allgemeinen Formel

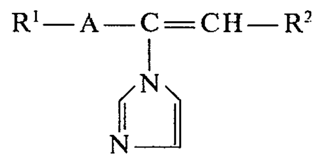

(I)

in welcher

A für die Ketogruppe oder eine CH(OH)-Gruppierung steht,

$R^1$ für tert.-butyl, Chlor-tert.-butyl, Fluor-tert.-butyl, Dichlor-tert.-butyl oder Difluor-tert.-butyl steht, und

$R^2$ für geradkettiges oder verzweigtes Alkyl mit 1 bis 29 Kohlenstoffatomen, für gegebenenfalls durch Alkyl mit 1 bis 4 Kohlenstoffatomen substituiertes Cycloalkyl und Cycloalkenyl mit jeweils 5 bis 7 Kohlenstoffatomen; für geradkettiges oder verzweigtes Halogenalkyl mit 1 bis 29, insbesondere 1 bis 18 Kohlenstoffatomen und 1 bis 5 gleichen oder verschiedenen Halogenatomen; für Alkoxyalkyl und Alkylmercaptoalkyl mit jeweils 1 bis 4 Kohlenstoffatomen in jedem Alkylteil; für geradkettiges oder verzweigtes Dialkylaminoalkyl und 1 bis 4 Kohlenstoffatomen in den Alkylresten der Aminogruppe und 1 bis 29, insbesondere 1 bis 18 Kohlenstoffatomen im Alkylteil; für geradkettiges oder verzweigtes Alkenyl, Alkinyl und Alkeninyl mit jeweils bis zu 6 Kohlenstoffatomen, wobei als Substituenten infrage kommen: Hydroxy, Alkoxy mit 1 bis 4 Kohlenstoffatomen sowie Phenyl, das gegebenenfalls substituiert sein kann durch: Halogen, Alkyl mit 1 bis 4 Kohlenstoffatomen, Alkoxy und Alkylthio mit jeweils 1 bis 4 Kohlenstoffatomen, Halogenalkoxy und Halogenalkylthio mit jeweils 1 bis 5 gleichen oder verschiedenen Halogenatomen sowie Cyano und Nitro; für gegebenenfalls einfach oder mehrfach, gleich oder verschieden substituiertes Phenylalkyl mit 1 bis 4 Kohlenstoffatomen im Alkylteil, wobei als Phenylsubstituenten die bereits oben genannten infrage kommen und wobei als Alkylsubstituenten infrage kommen: Cyano, Hydroxycarbonyl und Alkoxycarbonyl mit 1 bis 4 Kohlenstoffatomen im Alkylteil; für gegebenenfalls durch Halogen, Alkyl und Alkoxy mit jeweils 1 bis 4 Kohlenstoffatomen substituiertes Indenyl und Fluorenyl; sowie für gegebenenfalls substituiertes Diphenyl- und Triphenylmethyl, wobei als Phenylsubstituenten die bereits oben genannten infrage kommen, steht, und/ oder deren physiologisch verträglichen Säureadditionssalze gute antimykotische Eigenschaften besitzen und zur Behandlung von Mykosen geeignet sind.

Die Verbindungen der allgemeinen Formel (I) können in zwei geometrischen Isomerenformen (E- und Z-Form) vorliegen, je nach Anordnung der Gruppen, die an die Doppelbindung gebunden sind; vorzugsweise fallen sie in einem wechselnden E,Z-Isomerenverhältnis an. Wenn A für die CH(OH)-Gruppierung steht, liegt ein asymmetrisches Kohlenstoffatom vor, so daß die Verbindungen der Formel (I) in diesem Fall außerdem in zwei optischen Isomerenformen anfallen, vorzugsweise fallen sie als Racemate an. Die vorliegende Erfindung betrifft sowohl die einzelnen Isomeren als auch die Isomeren-Gemische.

Überraschenderweise zeigen die erfindungsgemäßen Imidazolyl-vinyl-ketone und -carbinole ein allgemein besseres antimykotisches Wirkungsspektrum, insbesondere gegen Sproßpilze und Schimmelpilze, und insbesondere eine bessere in-vivo-Wirkung als die aus dem Stand der Technik bekannten 1-Phenyl-2-imidazolyl-4,4-dimethyl-1-penten-3-one und -ole, welche chemisch sehr naheliegende Verbindungen sind. Die erfindungsgemäße Verwendung der neuen Stoffe stellt somit eine Bereicherung der Pharmazie dar.

Im einzelnen seien außer den bei den Herstellungsbeispielen genannten Verbindungen die folgenden Verbindungen der allgemeinen Formel (I) genannt:

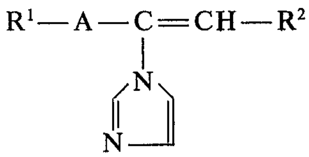 (I)

| $R^1$ | $R^2$ | A |
|---|---|---|
| $(CH_3)_3C-$ | $-CH(C_2H_5)_2$ | CO |
| $(CH_3)_3C-$ | $-C(CH_3)_3$ | CO |
| $(CH_3)_3C-$ | 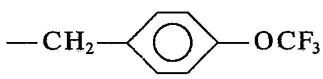 | CO |
| $(CH_3)_3C-$ | 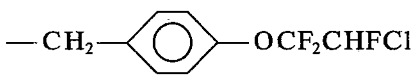 | CO |
| $(CH_3)_3C-$ | $-CH_2-CH_2-O-C_3H_7\text{-n}$ | CO |
| $(CH_3)_3C-$ | $-C_{23}H_{27}\text{-n}$ | CO |
| $(CH_3)_3C-$ | $-CH_2-CH_2-S-C_3H_7\text{-n}$ | CO |
| $(CH_3)_3C-$ | $-(CH_2)_3-N(CH_3)_2$ | CO |
| $(CH_3)_3C-$ | $-(CH_2)_7-OH$ | CO |
| $(CH_3)_3C-$ | 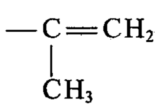 | CO |
| $(CH_3)_3C-$ | 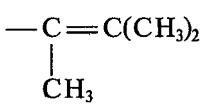 | CO |
| $(CH_3)_3C-$ | $-(CH_2)_7F$ | CO |
| $(CH_3)_3C-$ | $-(CH_2)_7Cl$ | CO |
| $(CH_3)_3C-$ | 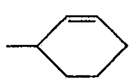 | CO |
| $(CH_3)_3C-$ | 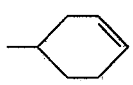 | CO |

Fortsetzung

| $R^1$ | $R^2$ | A |
|---|---|---|
| $ClCH_2—C(CH_3)_2—$ | $—C_2H_5$ | CO |
| $ClCH_2—C(CH_3)_2—$ | $—CH(CH_3)(C_2H_5)$ | CO |
| $ClCH_2—C(CH_3)_2—$ | $—C_4H_9$-n | CO |
| $ClCH_2—C(CH_3)_2—$ | $—CH(CH_3)_2$ | CO |
| $ClCH_2—C(CH_3)_2—$ | $—C(CH_3)_3$ | CO |
| $ClCH_2—C(CH_3)_2—$ | 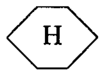 | CO |
| $ClCH_2—C(CH_3)_2—$ | $—CH_2—$ 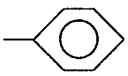 | CO |
| $ClCH_2—C(CH_3)_2—$ | $—C\equiv C—$ 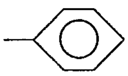 | CO |
| $FCH_2—C(CH_3)_2—$ | $—C_2H_5$ | CO |
| $FCH_2—C(CH_3)_2—$ | $—C_4H_9$-n | CO |
| $FCH_2—C(CH_3)_2—$ | $—C(CH_3)_3$ | CO |
| $FCH_2—C(CH_3)_2—$ | $—C_7H_{15}$-n | CO |
| $FCH_2—C(CH_3)_2—$ | 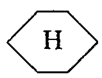 | CO |
| $FCH_2—C(CH_3)_2—$ | $—CH_2—$ 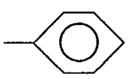 | CO |
| $FCH_2—C(CH_3)_2—$ | $—C\equiv C—$ 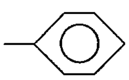 | CO |
| $CH_3—C(CH_2Cl)_2—$ | $—C_2H_5$ | CO |
| $CH_3—C(CH_2Cl)_2—$ | $—CH(CH_3)(C_2H_5)$ | CO |
| $CH_3—C(CH_2Cl)_2—$ | $—C_4H_9$-n | CO |
| $CH_3—C(CH_2Cl)_2—$ | $—CH(CH_3)_2$ | CO |
| $CH_3—C(CH_2Cl)_2—$ | $—C(CH_3)_3$ | CO |
| $CH_3—C(CH_2Cl)_2—$ | $—C_7H_{15}$-n | CO |
| $CH_3—C(CH_2Cl)_2—$ | 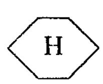 | CO |
| $CH_3—C(CH_2Cl)_2—$ | $—CH_2—$ 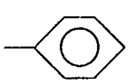 | CO |
| $CH_3—C(CH_2Cl)_2—$ | $—C\equiv C—$ 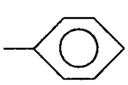 | CO |

Fortsetzung

| $R^1$ | $R^2$ | A |
|---|---|---|
| $CH_3-C(CH_2F)_2-$ | $-C_2H_5$ | CO |
| $CH_3-C(CH_2F)_2-$ | $-CH(CH_3)(C_2H_5)$ | CO |
| $CH_3-C(CH_2F)_2-$ | $-C_4H_9$-n | CO |
| $CH_3-C(CH_2F)_2-$ | $-CH(CH_3)_2$ | CO |
| $CH_3-C(CH_2F)_2-$ | $-C(CH_3)_3$ | CO |
| $CH_3-C(CH_2F)_2-$ | $-C_7H_{15}$-n | CO |
| $CH_3-C(CH_2F)_2-$ | 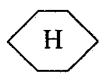 | CO |
| $CH_3-C(CH_2F)_2-$ | $-CH_2-$ 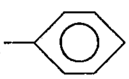 | CO |
| $CH_3-C(CH_2F)_2-$ | $-C{\equiv}C-$ 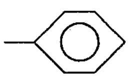 | CO |
| $(CH_3)_3C-$ | $-CH(C_2H_5)_2$ | CH(OH) |
| $(CH_3)_3C-$ | $-C(CH_3)_3$ | CH(OH) |
| $(CH_3)_3C-$ | $-CH_2-$ 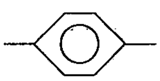 $-OCF_3$ | CH(OH) |
| $(CH_3)_3C-$ | $-CH_2-$ 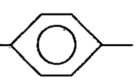 $-OCF_2CHFCl$ | CH(OH) |
| $(CH_3)_3C-$ | $-CH_2-CH_2-O-C_3H_7$-n | CH(OH) |
| $(CH_3)_3C-$ | $-C_{23}H_{47}$-n | CH(OH) |
| $(CH_3)_3C-$ | $-CH_2-CH_2-S-C_3H_7$-n | CH(OH) |
| $(CH_3)_3C-$ | $-(CH_2)_3-N(CH_3)_2$ | CH(OH) |
| $(CH_3)_3C-$ | $-(CH_2)_7-OH$ | CH(OH) |
| $(CH_3)_3C-$ | $-C(CH_3){=}CH_2$ | CH(OH) |
| $(CH_3)_3C-$ | $-C(CH_3){=}C(CH_3)_3$ | CH(OH) |
| $(CH_3)_3C-$ | $-(CH_2)_7F$ | CH(OH) |
| $(CH_3)_3C-$ | $-(CH_2)_7Cl$ | CH(OH) |
| $(CH_3)_3C-$ | 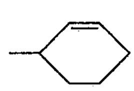 | CH(OH) |

Fortsetzung

| $R^1$ | $R^2$ | A |
|---|---|---|
| $(CH_3)_3C-$ | 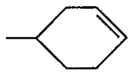 | CH(OH) |
| $ClCH_2-C(CH_3)_2-$ | $-C_2H_5$ | CH(OH) |
| $ClCH_2-C(CH_3)_2-$ | $-CH(CH_3)(C_2H_5)$ | CH(OH) |
| $ClCH_2-C(CH_3)_2-$ | $-C_4H_9$-n | CH(OH) |
| $ClCH_2-C(CH_3)_2-$ | $-CH(CH_3)_2$ | CH(OH) |
| $ClCH_2-C(CH_3)_2-$ | $-C(CH_3)_3$ | CH(OH) |
| $ClCH_2-C(CH_3)_2-$ | 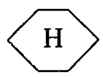 | CH(OH) |
| $ClCH_2-C(CH_3)_2-$ | $-CH_2-$ 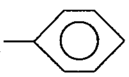 | CH(OH) |
| $ClCH_2-C(CH_3)_2-$ | $-C\equiv C-$ 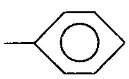 | CH(OH) |
| $FCH_2-C(CH_3)_2-$ | $-C_2H_5$ | CH(OH) |
| $FCH_2-C(CH_3)_2-$ | $-C_4H_9$-n | CH(OH) |
| $FCH_2-C(CH_3)_2-$ | $-C(CH_3)_3$ | CH(OH) |
| $FCH_2-C(CH_3)_2-$ | $-C_7H_{15}$-n | CH(OH) |
| $FCH_2-C(CH_3)_2-$ | 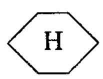 | CH(OH) |
| $FCH_2-C(CH_3)_2-$ | $-CH_2-$ 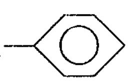 | CH(OH) |
| $FCH_2-C(CH_3)_2-$ | $-C\equiv C-$ 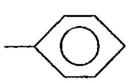 | CH(OH) |
| $CH_3-C(CH_2Cl)_2-$ | $-C_2H_5$ | CH(OH) |
| $CH_3-C(CH_2Cl)_2-$ | $-CH(CH_3)(C_2H_5)$ | CH(OH) |
| $CH_3-C(CH_2Cl)_2-$ | $-C_4H_9$-n | CH(OH) |
| $CH_3-C(CH_2Cl)_2-$ | $-CH(CH_3)_2$ | CH(OH) |
| $CH_3-C(CH_2Cl)_2-$ | $-C(CH_3)_3$ | CH(OH) |
| $CH_3-C(CH_2Cl)_2-$ | $-C_7H_{15}$-n | CH(OH) |
| $CH_3-C(CH_2Cl)_2-$ | 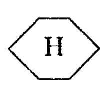 | CH(OH) |
| $CH_3-C(CH_2Cl)_2-$ | $-CH_2-$ 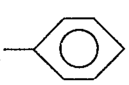 | CH(OH) |

Fortsetzung

| $R^1$ | $R^2$ | A |
|---|---|---|
| $CH_3—C(CH_2Cl)_2—$ | 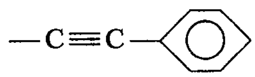 | CH(OH) |
| $(CH_3)_3C—$ | 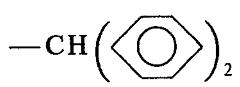 | CH(OH) |
| $(CH_3)_3C—$ | 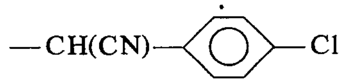 | CH(OH) |

Die erfindungsgemäß zu verwendenden Wirkstoffe sind noch nicht bekannt. Sie können jedoch gemäß einem eigenen Vorschlag hergestellt werden, indem man Ketoenamine der Formel

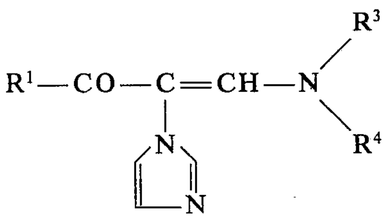

(II)

in welcher

$R^1$ die oben angegebene Bedeutung hat und
$R^3$ und $R^4$ gleich oder verschieden sind und für Alkyl stehen,

mit magnesium-organischen Verbindungen der Formel

$$Hal—Mg—R^2 \qquad (III)$$

in welcher

$R^2$ die oben angegebene Bedeutung hat und
Hal für Halogen steht,

in Gegenwart eines inerten organischen Lösungsmittels, wie beispielsweise Ether, und gegebenenfalls in Gegenwart eines Inertgases, wie beispielsweise Stickstoff, bei Temperaturen zwischen −20 und +120°C in üblicher Weise umsetzt und die entstehenden Keto-Derivate der Formel (I) gegebenenfalls nach bekannten Methoden reduziert, wie z. B. durch Umsetzung mit komplexen Hydriden, wie insbesondere Natriumborhydrid, gegebenenfalls in Gegenwart eines polaren Lösungsmittels, wie beispielsweise Alkohole, bei Temperaturen zwischen 0 und 30°C; oder durch Umsetzung mit Aluminiumisopropylat in Gegenwart eines polaren organischen Lösungsmittels, wie beispielsweise Isopropanol, bei Temperaturen zwischen 20 und 120°C. Die Aufarbeitung erfolgt in üblicher Weise; gegebenenfalls wird das Salz hergestellt. In manchen Fällen erweist es sich als vorteilhaft, die Verbindungen der Formel (I) über ihre Salze in reiner Form zu erhalten.

Die Ketoenamine der Formel (II) sind noch nicht bekannt; sie sind jedoch Gegenstand einer eigenen älteren Anmeldung, (vergleiche DE-A 3 000 643). Die Ketoenamine der Formel (II) können nach den dort beschriebenen Verfahren erhalten werden, indem man Imidazolyl-ketone der Formel

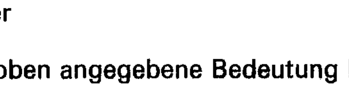

(IV)

in welcher

$R^1$ die oben angegebene Bedeutung hat,

mit Amidacetalen bzw. Aminalestern der Formeln

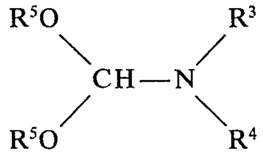

(V a)

bzw.

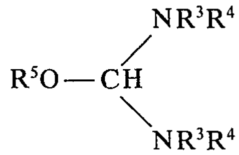

(V b)

in welchen

$R^3$ und $R^4$ die oben angegebene Bedeutung haben und
$R^5$ für Alkyl mit 1 bis 4 Kohlenstoffatomen steht,

in an sich bekannter Art und Weise in Gegenwart eines inerten organischen Lösungsmittels, wie beispielsweise eines aromatischen Kohlenwasserstoffs oder insbesondere eines Überschusses an eingesetztem Amidacetal bzw. Aminalester der Formel (V a) bzw. (V b), in der Siedehitze umsetzt (vergleiche hierzu auch Chem. Ber. 101, 41–50 [1968]; J. Org. Chem. 43, 4148–50 [1978] sowie die Herstellungsbeispiele).

Die Imidazolyl-ketone der Formel (IV) sind bekannt (vergleiche z. B. 2 610 022 und DE-OS 2 638 470); bzw. lassen sie sich nach üblichen Methoden herstellen, indem man die entsprechenden Halogen-ketone in Gegenwart eines Säurebinders mit Imidazol umsetzt.

Die Amidacetale bzw. Aminalester der Formeln (V a) bzw. (V b) sind allgemein bekannte Verbindungen der organischen Chemie (vergleiche z. B. Chem. Ber. 101, 41–50 [1968] und J. Org. Chem. 43, 4248–50 [1978]).

Die magnesium-organischen Verbindungen der Formel (III) sind allgemein bekannte Verbindungen der organischen Chemie.

Zur Herstellung von physiologisch verträglichen Säureadditionssalzen der Verbindungen der Formel (I) kommen vorzugsweise folgende Säuren infrage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäure, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der Formel (I) und ihre Säureadditionssalze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bisphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden: Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden: Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe

enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabellen, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnußöl, Maiskeimöl, Olivenöl, Rizinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

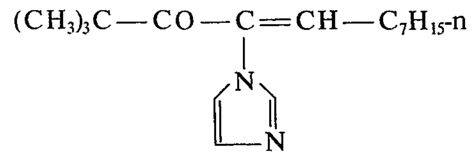

Zu 55,5 g (0,25 Mol) 4,4-Dimethyl-1-dimethylamino-2-(imidazol-1-yl)-pent-1-en-3-on in 800 ml Ether gibt man bei −20 °C innerhalb von 30 Minuten unter Inertgas (Stickstoff) eine Lösung von 61 g (0,3 Mol) n-Heptylmagnesiumbromid in 130 ml Ether. Nach beendeter Zugabe läßt man das Reaktionsgemisch innerhalb von ca. 2 Stunden auf Raumtemperatur erwärmen. Danach wird mit verdünnter Salzsäure versetzt, die organische Phase wird abgetrennt, mit Wasser gewaschen, über Natriumsulfat getrocknet und eingeengt. Man erhält 54 g (78 % der Theorie) 2,2-Dimethyl-4-(imidazol-1-yl)-5-dodecen-3-on vom Brechungsindex $n_D^{20} = 1,4802$.

### Herstellung des Ausgangsproduktes

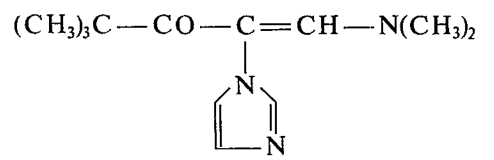

41,6 g (0,25 Mol) 3,3-Dimethyl-1-(imidazol-1-yl)-butan-2-on werden mit 35,7 g (0,3 Mol) Dimethylformamid-dimethylacetal 5 Stunden unter Rückfluß erhitzt. Danach wird das überschüssige Acetal abdestilliert. Das zurückbleibende Öl kristallisiert beim Abkühlen. Man erhält 50 g (90,5 % der Theorie) 4,4-Dimethyl-1-dimethylamino-2-(imidazol-1-yl)-pent-1-en-3-on vom Schmelzpunkt 45–50 °C.

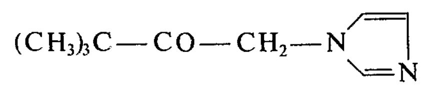

136,2 g (2 Mol) Imidazol werden bei Raumtemperatur portionsweise zu 276,4 g (2 Mol) gemahlenem Kaliumcarbonat und 269,2 g (2 Mol) $\alpha$-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand wird destilliert. Man erhält 296 g (89 % der Theorie) 3,3-Dimethyl-1-(imidazol-1-yl)-butan-2-on vom Schmelzpunkt ~20 °C.

Beispiel 2

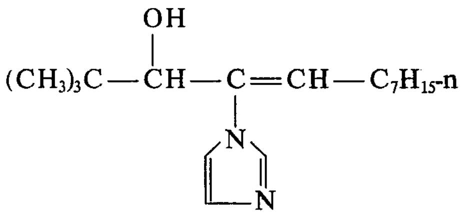

(Reduktion)

Zu 27,6 g (0,1 Mol) 2,2-Dimethyl-4-(imidazol-1-yl)-5-dodecen-3-on (Beispiel 1) in 100 ml Methanol werden bei 0 °C 1,1 g (0,029 Mol) Natriumborhydrid, in 10 ml Wasser gelöst, getropft. Nach beendeter Zugabe läßt man 2 Stunden bei Raumtemperatur nachrühren. Danach wird mit verdünnter Salzsäure ein pH-Wert von 6–7 eingestellt, eingeengt und der Rückstand mit Chloroform extrahiert. Die organische Phase wird über Natriumsulfat getrocknet und eingeengt. Man erhält 25,6 g (92 % der Theorie) 2,2-Dimethyl-4-(imidazol-1-yl)-5-decen-3-ol vom Brechungsindex $n_D^{20} = 1,4860$.

Analog werden die folgenden Verbindungen der allgemeinen Formel (I)

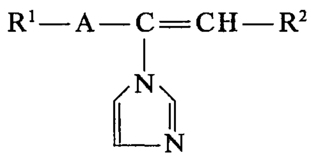

(I)

erhalten:

| Bsp. Nr. | $R^1$ | $R^2$ | A | Schmelzpunkt (°C) bzw. Brechungs-index $n_D^{20}$ |
|---|---|---|---|---|
| 3 | $(CH_3)_3C-$ | $-CH_3$ | CO | 1,5097 |
| 4 | $(CH_3)_3C-$ | $-C_2H_5$ | CO | 1,5032 |
| 5 | $(CH_3)_3C-$ | $-CH(CH_3)_2$ | CO | 1,4928 |
| 6 | $(CH_3)_3C-$ | $-C_4H_9$-n | CO | 1,4926 |
| 7 | $(CH_3)_3C-$ | $-C(CH_3)_3$ | CO | zähfl. Öl |
| 8 | $(CH_3)_3C-$ | $-CH(CH_3)C_2H_5$ | CO | 30 |
| 9 | $(CH_3)_3C-$ | $-CH{=}CH_2$ | CO | zähfl. Öl |
| 10 | $(CH_3)_3C-$ | 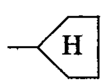 | CO | 1,5121 |
| 11 | $(CH_3)_3C-$ | 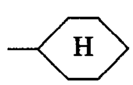 | CO | 1,5005 |
| 12 | $(CH_3)_3C-$ | $-CH_2-$ 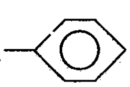 | CO | 1,5430 |
| 13 | $(CH_3)_3C-$ | $-C{\equiv}C-$ 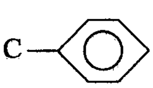 | CO | zähfl. Öl |

Fortsetzung

| Bsp. Nr. | $R^1$ | $R^2$ | A | Schmelzpunkt (°C) bzw. Brechungsindex $n_D^{20}$ |
|---|---|---|---|---|
| 14 | $(CH_3)_3C-$ | —CH 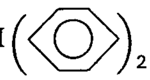 | CO | 35–40 |
| 15 | $(CH_3)_3C-$ | $-C_8H_{17}$-n | CO | 1,4759 |
| 16 | $(CH_3)_3C-$ | $-C_9H_{19}$-n | CO | 1,4769 |
| 17 | $(CH_3)_3C-$ | $-C_{10}H_{21}$-n | CO | 1,4820 |
| 18 | $(CH_3)_3C-$ | $-C_6H_{13}$-n | CO | 1,4835 |
| 19 | $(CH_3)_3C-$ | $-C_{12}H_{25}$-n | CO | 1,4820 |
| 20 | $FCH_2-C(CH_3)_2-$ | $-CH(CH_3)C_2H_5$ | CO | 1,4947 |
| 21 | $FCH_2-C(CH_3)_2-$ | $-CH(CH_3)_2$ | CO | 1,5100 |
| 22 | $(CH_3)_3C-$ | 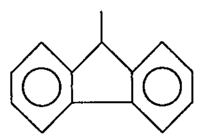 | CO | 186–87 |
| 23 | $(CH_3)_3C-$ | —CH(CN)— 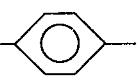 —Cl | CO | 177–79 |
| 24 | $(CH_3)_3C-$ | $-C_4H_9$-n | CH(OH) | 1,4970 |
| 25 | $(CH_3)_3C-$ | $-CH_3$ | CH(OH) | 1,5106 |
| 26 | $(CH_3)_3C-$ | $-CH(CH_3)_2$ | CH(OH) | 1,4979 |
| 27 | $(CH_3)_3C-$ | $-CH(CH_3)C_2H_5$ | CH(OH) | 1,4966 |
| 28 | $(CH_3)_3C-$ | 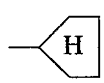 | CH(OH) | 1,5100 |
| 29 | $(CH_3)_3C-$ | $-C(CH_3)_3$ | CH(OH) | 30 |
| 30 | $(CH_3)_3C-$ | $-CH_2-$ 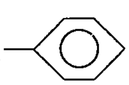 | CH(OH) | 117–25 |
| 31 | $(CH_3)_3C-$ | $-C\equiv C-$ 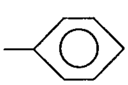 | CH(OH) | 1,5485 |
| 32 | $(CH_3)_3C-$ | 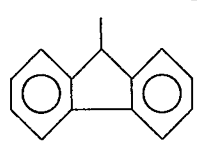 | CH(OH) | 199–201 |
| 33 | $(CH_3)_3C-$ | $-C_8H_{17}$-n | CH(OH) | 1,4875 |
| 34 | $(CH_3)_3C-$ | $-C_9H_{19}$-n | CH(OH) | 1,4809 |
| 35 | $(CH_3)_3C-$ | $-C_{12}H_{25}$-n | CH(OH) | 1,4860 |

Fortsetzung

| Bsp. Nr. | $R^1$ | $R^2$ | A | Schmelzpunkt (°C) bzw. Brechungs-index $n_D^{20}$ |
|---|---|---|---|---|
| 36 | $(CH_3)_3C—$ | $—C_6H_{27}$-n | CH(OH) | 1,4837 |
| 37 | $(CH_3)_3C—$ | 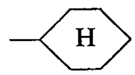 | CH(OH) | 1,5100 |
| 38 | $(CH_3)_3C—$ | $—C_2H_5$ | CH(OH) | 1,5120 |
| 39 | $(CH_3)_3C—$ | $—C_{10}H_{21}$-n | CH(OH) | 1,4800 |
| 40 | $(CH_3)_3C—$ | $—C_{18}H_{37}$-n | CO | Öl |
| 41 | $(CH_3)_3C—$ | $—CH_2—CH(CH_3)_2$ | CH(OH) | 1,4925 |
| 42 | $(CH_3)_3C—$ | $—CH_2CH_2CH(CH_3)_2$ | CH(OH) | 1,4875 |
| 43 | $CH_3—C(CH_2F)_2—$ | $CH_3$ | CH(OH) | 25 |
| 44 | $CH_3—C(CH_2F)_2—$ | $—CH(CH_3)—C_2H_5$ | CH(OH) | Öl |
| 45 | $(CH_3)_3C—$ | $—CH_2—CH(CH_3)_2$ | CO | 1,4937 |
| 46 | $(CH_3)_3C—$ | $—CH_2CH_2CH(CH_3)_2$ | CO | 1,4893 |
| 47 | $CH_3—C(CH_2F)_2—$ | $—CH(CH_3)—C_2H_5$ | CO | Öl |
| 48 | $CH_3—C(CH_2F)_2—$ | $CH_3$ | CO | Öl |
| 49 | $CH_3—C(CH_2F)_2—$ | $—CH(CH_3)_2$ | CO | Öl |

Verwendungsbeispiele

In den nachfolgenden Beispielen werden die nachstehend angegebenen Verbindungen als Vergleichssubstanzen eingesetzt:

(A) 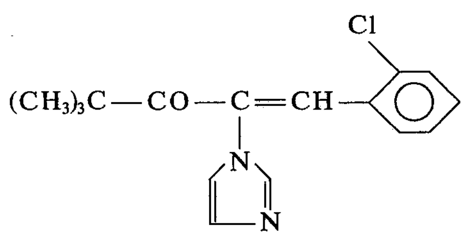

(B) 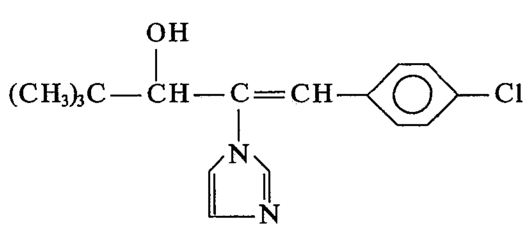

## Beispiel A

### Antimykotische in-vitro-Wirksamkeit

### Versuchsbeschreibung

Die in -vitro-Wirksamkeit im Reihenverdünnungstest wird mit Keiminokula von durchschnittlich $5 \times 10^4$ Keimen/ml Substanz durchgeführt. Als Nährmedium dienten:

a) für Dermatophyten und Schimmelpilze:
   Sabouraud's milieu d'épreuve

b) für Hefen:
   Isotonic Sensitest-Broth von Oxid.

Die Bebrütungstemperatur betrug 28°C; Bebrütungsdauer war 24 Stunden bei Hefen und 96 Stunden bei Dermatophyten und Schimmelpilzen.
In diesem Test zeigen insbesondere die Beispiele 1, 2, 5, 7, 8, 10, 28, 32 und 37 eine bessere antimykotische Wirkung als die aus dem Stand der Technik bekannten Verbindung (A).

## Beispiel B

### Antimykotische in-vivo-Wirksamkeit (oral) bei Mäuse-Candidoese

### Versuchsbeschreibung

Mäuse vom Typ SPF-$CF_1$ wurden intravenös mit $1-2 \times 10^6$ logarithmisch wachsenden Candida-Zellen, die in physiologischer Kochsalzlösung suspendiert waren, infiziert. Eine Stunde vor und sieben Stunden nach der Infektion werden die Tiere mit jeweils 50—100 mg/kg Körpergewicht der Präparate oral behandelt.

### Ergebnis

Unbehandelte Tiere starben 3 bis 6 Tage post infektionem. Die Überlebensrate am 6. Tag post infektionem betrug bei unbehandelten Kontrolltieren etwa 5 %.
In diesem Test zeigen insbesondere die Beispiele 5, 7, 8 und 25 gute bis sehr gute Wirkung, während die aus dem Stand der Technik bekannten Verbindungen (A) und (B) keine Wirkung zeigen.

## Beispiel C

### Antimikrobielle in-vivo-Wirksamkeit (lokal) am Modell der experimentellen Meerschweinchen-Trichophytie

### Versuchsbeschreibung

Weiße Meerschweinchen der Rasse Pirbright-white wurden auf dem geschorenen, nicht skarifizierten Rücken mit einer Mikro- und Makrokonidien-Suspension von Trichophyton mentagrophytes infiziert.
Die infizierten Tiere wurden, beginnend am 3. Tag p. i., 1 × täglich mit 1%iger Lösung der erfindungsgemäßen Präparate (in Dimethylsulfoxid : Glycerin = 1 : 4) lokal behandelt.

### Ergebnis

Bei unbehandelten Tieren entwickelt sich innerhalb von 12 Tagen p. i. das typische Bild einer Dermatophytose mit Rötung, Schuppung und Haarausfall bis zum totalen Integument-Defekt an der Infektionsstelle.
Dagegen zeigen z. B. die erfindungsgemäßen Beispiele 1, 2, 8 und 27 Wirkung bis gute Wirkung, d. h. lediglich geringe Rötung und vereinzelt Schuppen.

**Patentansprüche**

1 Imidazolyl-vinyl-ketone und/oder Carbinole der allgemeinen Formel

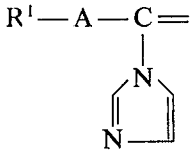

(I)

in welcher

A für die Ketogruppe oder eine CH(OH)-Gruppierung steht,
$R^1$ für tert.-Butyl, Chlor-tert.-butyl, Fluor-tert.-butyl, Dichlor-tert.-butyl oder Difluor-tert.-butyl steht, und
$R^2$ geradkettiges oder verzweigtes Alkyl mit 1 bis 29 Kohlenstoffatomen, für gegebenenfalls durch Alkyl mit 1 bis 4 Kohlenstoffatomen substituiertes Cycloalkyl und Cycloalkenyl mit jeweils 5 bis 7 Kohlenstoffatomen; für geradkettiges oder verzweigtes Halogenalkyl mit 1 bis 29, insbesondere 1 bis 18 Kohlenstoffatomen und 1 bis 5 gleichen oder verschiedenen Halogenatomen; für Alkoxyalkyl und Alkylmercaptoalkyl mit jeweils 1 bis 4 Kohlenstoffatomen in jedem Alkylteil; für geradkettiges oder verzweigtes Dialkylaminoalkyl und 1 bis 4 Kohlenstoffatomen in den Alkylresten der Aminogruppe und 1 bis 29, insbesondere 1 bis 18 Kohlenstoffatomen; für gegebenenfalls substituiertes, geradkettiges oder verzweigtes Alkenyl, Alkinyl und Alkeninyl mit jeweils bis 6 Kohlenstoffatomen, wobei als Substituenten infrage kommen: Hydroxy, Alkoxy mit 1 bis 4 Kohlenstoffatomen sowie Phenyl, das gegebenenfalls substituiert sein kann durch: Halogen, Alkyl mit 1 bis 4 Kohlenstoffatomen, Alkoxy und Alkylthio mit jeweils 1 bis 4 Kohlenstoffatomen, Halogenalkoxy und Halogenalkylthio mit jeweils 1 bis 5 gleichen oder verschiedenen Halogenatomen sowie Cyano und Nitro; für gegebenenfalls einfach oder mehrfach, gleich oder verschieden substituiertes Phenylalkyl mit 1 bis 4 Kohlenstoffatomen im Alkylteil, wobei als Phenylsubstituenten die bereits oben genannten infrage kommen und wobei als Alkylsubstituenten infrage kommen: Cyano, Hydroxycarbonyl und Alkoxycarbonyl mit 1 bis 4 Kohlenstoffatomen im Alkylteil; für gegebenenfalls durch Halogen, Alkyl und Alkoxy mit jeweils 1 bis 4 Kohlenstoffatomen substituiertes Indenyl und Fluorenyl; sowie für gegebenenfalls substituiertes Diphenyl- und Triphenylmethyl, wobei als Phenylsubstituenten die bereits oben genannten infrage kommen, steht, und/oder deren physiologisch verträglichen Säureadditionssalze zur Behandlung von Mykosen.

2. 2,2-Dimethyl-4-(imidazol-1-yl)-5-dodecen-3-on zur Behandlung von Mykosen.
3. 2,2-Dimethyl-4-(imidazol-1-yl)-5-decen-3-ol zur Behandlung von Mykosen.
4. 2,2-Dimethyl-4-(imidazol-1-yl)-5-hexen-3-on zur Behandlung von Mykosen.
5. 2,2-Dimethyl-4-(imidazol-1-yl)-5-hepten-3-on zur Behandlung von Mykosen.
6. Verfahren zur Herstellung eines antimykotischen Mittels, dadurch gekennzeichnet, daß man Imidazolyl-vinyl-ketone oder -carbinole gemäß der Formel in Anspruch 1 mit inerten, nichttoxischen physiologisch verträglichen Trägerstoffen vermischt.
7. Verfahren zur Herstellung eines antimykotischen Mittels, dadurch gekennzeichnet, daß man physiologisch verträgliche Säureadditionssalze von Imidazolyl-vinyl-ketonen oder -carbinolen gemäß der Formel in Anspruch 1 mit inerten, nichttoxischen physiologisch verträglichen Trägerstoffen vermischt.
8. Verfahren zur Herstellung eines antimykotischen Mittels, dadurch gekennzeichnet, daß man Imidazolyl-vinyl-ketone und/oder -carbinole und/oder deren physiologisch verträglichen Säureadditionssalze in einem geeigneten inerten Lösungsmittel, gegebenenfalls unter Hinzufügung einer Säure, löst.

**Claims**

1. Imidazolyl-vinyl ketones and/or carbinols of the general formula

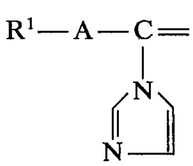

(I)

in which

A represents the keto group or a CH(OH) grouping,

$R^1$ represents tert.-butyl, chloro-tert.-butyl, fluoro-tert.-butyl, dichloro-tert.-butyl or difluoro-tert.-butyl, and

$R^2$ represents straight-chain or branched alkyl with 1 to 29 carbon atoms, cycloalkyl or cycloalkenyl which has in each case 5 to 7 carbon atoms and is optionally substituted by alkyl with 1 to 4 carbon atoms; straight-chain or branched halogenoalkyl with 1 to 29, in particular 1 to 18, carbon atoms and 1 to 5 identical or different halogen atoms; alkoxyalkyl or alkylmercaptoalkyl with in each case 1 to 4 carbon atoms in each alkyl part; straight-chain or branched dialkylaminoalkyl and 1 to 4 carbon atoms in the alkyl radicals of the amino group and 1 to 29, in particular 1 to 18, carbon atoms; optionally substituted, straight-chain or branched alkenyl, alkynyl or alkenynyl with in each case up to 6 carbon atoms, possible substituents being: hydroxyl, alkoxy with 1 to 4 carbon atoms and phenyl which can optionally be substituted by: halogen, alkyl with 1 to 4 carbon atoms, alkoxy or alkylthio with in each case 1 to 4 carbon atoms, halogenoalkoxy or halogenoalkylthio with in each case 1 to 5 identical or different halogen atoms as well as cyano or nitro; phenylalkyl which has 1 to 4 carbon atoms in the alkyl part and is optionally monosubstituted or polysubstituted by identical or different substituents, possible substituents on the phenyl being those already mentioned above, and possible substituents on the alkyl being: cyano, hydroxycarbonyl and alkoxycarbonyl with 1 to 4 carbon atoms in the alkyl part; indenyl or fluorenyl which is optionally substituted by halogen, or by alkyl or alkoxy with in each case 1 to 4 carbon atoms; or optionally substituted diphenyl- or triphenyl-methyl, possible substituents on the phenyl being those already mentioned above, and/or physiologically acceptable acid addition salts thereof, for the treatment of mycoses.

2. 2,2-Dimethyl-4-(imidazol-1-yl)-5-dodecen-3-one for the treatment of mycoses.
3. 2,2-Dimethyl-4-(imidazol-1-yl)-5-decen-3-ole for the treatment of mycoses.
4. 2,2-Dimethyl-4-(imidazol-1-yl)-5-hexen-3-one for the treatment of mycoses.
5. 2,2-Dimethyl-4-(imidazol-1-yl)-5-hepten-3-one for the treatment of mycoses.
6. Process for the preparation of an antimycotic agent, characterised in that imidazolyl-vinyl ketones or carbinols according to the formula in Claim 1 are mixed with inert, non-toxic physiologically acceptable excipients.
7. Process for the preparation of an antimycotic agent, characterised in that physiologically acceptable acid addition salts of imidazolyl-vinyl ketones or carbinols according to the formula in Claim 1 are mixed with inert, non-toxic physiologically acceptable excipients.
8. Process for the preparation of an antimycotic agent, characterised in that imidazolyl-vinyl ketones and/or carbinols and/or physiologically acceptable acid addition salts thereof are dissolved in a suitable inert solvent, an acid being added if appropriate.

**Revendications**

1. Imidazolyl-vinyl-cétones et/ou -carbinols de formule générale

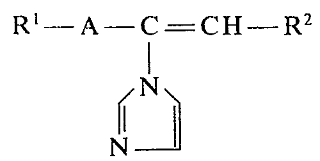

(I)

dans laquelle

A représente le groupe céto ou un groupement CH(OH),

$R^1$ est un groupe tertio-butyle, chloro-tertio-butyle, fluoro-tertio-butyle, dichloro-tertio-butyle ou difluoro-tertio-butyle, et

$R^2$ est un groupe alkyle à chaîne droite ou ramifié ayant 1 à 29 atomes de carbone, un groupe cycloalkyle et un groupe cycloalcényle ayant chacun 5 à 7 atomes de carbone, éventuellement substitués par un groupe alkyle ayant 1 à 4 atomes de carbone; un groupe halogénalkyle à chaîne droite ou ramifié ayant 1 à 29, notamment 1 à 18 atomes de carbone et 1 à 5 atomes d'halogènes égaux ou différents; un groupe alkoxyalkyle et un groupe alkylmercaptoalkyle ayant chacun 1 à 4 atomes de carbone dans chaque partie alkyle; un groupe dialkylaminoalkyle à chaîne droite ou ramifié ayant 1 à 4 atomes de carbone dans les restes alkyle du groupe amino et 1 à 29, notamment 1 à 18 atomes de carbone; un groupe alcényle, alcynyle et alcéninyle à chaîne droite ou ramifié, éventuellement substitué, ayant chacun jusqu'à 6 atomes de carbone, les substituants que l'on considère étant les suivants: hydroxy, alkoxy ayant 1 à 4 atomes de carbone ainsi que phényle qui peut être substitué, le cas échéant, par: un halogène, un radical alkyle ayant 1 à 4 atomes de carbone, un radical alkoxy et un radical alkylthio ayant chacun 1 à 4 atomes de carbone, un radical halogénalkoxy et un radical halogénalkylthio ayant chacun 1 à 5 atomes d'halogènes

égaux ou différents ainsi que le radical cyano et le radical nitro; un groupe phénylalkyle portant éventuellement un ou plusieurs substituants égaux ou différents, ayant 1 à 4 atomes de carbone dans la partie alkyle, les substituants de la partie phényle étant ceux qui ont déjà été mentionnés ci-dessus, et les substituants de la partie alkyle pouvant être les suivants: cyano, hydroxycarbonyle et alkoxycarbonyle ayant 1 à 4 atomes de carbone dans la partie alkyle; un groupe indényle et un groupe fluorényle éventuellement substitués par un halogène, un radical alkyle et un radical alkoxy ayant chacun 1 à 4 atomes de carbone; ainsi qu'un groupe diphénylméthyle et un groupe triphénylméthyle éventuellement substitués, en considérant alors comme substituants du groupe phényle ceux qui ont déjà été mentionnés ci-dessus, et/ou leurs sels d'addition d'acides physiologiquement acceptables, pour le traitement de mycoses.

2. La 2,2-diméthyl-4-(imidazole-1-yl)-5-dodécène-3-one destinée au traitement de mycoses.
3. Le 2,2-diméthyl-4-(imidazole-1-yl)-5-décène-3-ol destiné au traitement de mycoses.
4. La 2,2-diméthyl-4-(imidazole-1-yl)-5-hexène-3-one destinée au traitement de mycoses.
5. La 2,2-diméthyl-4-(imidazole-1-yl)-5-heptène-3-one destinée au traitement de mycoses.
6. Procédé de production d'une composition antimycosique, caractérisé en ce qu'on mélange des imidazolyl-vinyl-cétones ou -carbinols de formule suivant la revendication 1 avec des supports inerts non toxiques acceptables du point de vue physiologique.
7. Procédé de production d'une composition antimycosique, caractérisé en ce qu'on mélange des sels d'addition d'acides physiologiquement acceptables d'imidazolyl-vinyl-cétones ou -carbinols de formule suivant la revendication 1 avec des supports inerts non toxiques acceptables du point de vue physiologique.
8. Procédé de production d'une composition antimycosique, caractérisé en ce qu'on dissout des imidazolyl-vinyl-cétones et/ou -carbinols et/ou leurs sels d'addition d'acides physiologiquement acceptables dans un solvant inerte approprié, éventuellement avec addition d'un acide.